# EUROPEAN PATENT SPECIFICATION

(11) **EP 0 585 379 B1**
(45) Date of publication and mention of the grant of the patent: **30.09.1998**
(21) Application number: 92913029.2
(22) Date of filing: 12.05.1992
(51) Int. Cl.: A61M 11/00, A61M 15/00, B67D 5/06, B65D 83/00

(54) **AEROSOL INHALATION DEVICE**
AEROSOLINHALATIONSVORRICHTUNG
DISPOSITIF D'INHALATION EN AEROSOL

(30) Priority: 21.05.1991 US 703646
(43) Date of publication of application: 09.03.1994
(73) Proprietor: ABBOTT LABORATORIES, Abbott Park, Illinois 60064-3500 (US)
(72) Inventor: ADJEI, Akwete, L., Wadsworth, IL 60083 (US); SHERRY, Lois, R., Lincolnshire, IL 60069 (US); DANKS, Barbara, A., Greers Ferry, AR 72067 (US)
(74) Representative: Modiano, Guido, Dr.-Ing.
(86) International application number: US9203945
(87) International publication number: WO9220391

(56) References cited:
- GB-A- 1 128 655
- US-A- 3 739 950
- US-A- 3 994 421
- US-A- 4 641 644

## Description

This invention relates to a two-part aerosol inhalation device which maximizes the delivery of a medicament, such as LH-RH analog or other peptide, into the deepest part of the lung where it becomes most effective. With most inhalers of the type wherein a pressurized canister of medicament is mounted in a cylindrical holder, one end of which is intended to be pointed toward a patient's open mouth, the medicament is directed from the canister and through the cylindrical holder at such a high velocity that too high a percentage of the medicament merely impacts as relatively large particles against the back of the patient's throat with only a small percentage of the medicament finding its way into the deepest part of the patient's lung. Such pocket inhalers are well known in the art, including several which are of two-part construction, namely U.S. Patent Nos. 3,739,950; 3,927,806; 3,994,421; 4,130,116; and 4,637, 528.

Both US 3,994,421 and US 4,637,528 are directed to retractable medicament spray dispensers having sliding and pivoting joints which are designed in such a manner that incorrect assembly is avoided even when the user is under stress or adverse environmental conditions. GB 1,128,655 which is directed to a one-part inhaler, discloses an oval exterior configuration of the inhaler's mouthpiece to prevent the leakage of air around it. An aerosol inhalation device according to the preamble of claim 1 is disclosed in US-A-4,641,644.

However, none of these inhalators according to the prior art provides the effective and efficient medicament delivery results which are most beneficial to a patient having a lung disorder requiring maximization of the delivered dose to be deposited in the deepest portion of the lung for absorption into the circulatory system.

The present invention is directed to an improved design for an aerosol inhalation device which effectively delivers a larger percentage of a medicament, especially of a poorly-absorbed drug such as a peptide, to the deepest part of the patient's lungs than is delivered by currently available inhalers.

An object of the present invention is to provide an aerosol inhalation device according to the preamble of claims which is characterized by an elongated expansion chamber which is elliptical in cross section whereby the velocity of the medicament particles is sufficiently slowed down so that a greater amount there of is delivered into the deepest portion of the lungs rather than being impacted against the back of the throat.

This object is achieved by an aerosol inhalation device according to claim 1 of the present invention.

The invention itself together with the foregoing objects, features, and advantages thereof, and others, will be best understood by reference to the following detailed description taken in conjunction with the drawing, in which:
FIG. 1 is a top plan view of a preferred embodiment of an aerosol inhalation device embodying the invention when in its compact storage mode;
FIG. 2 is a top plan view of the device of Fig. 1 with the mouthpiece protective cap removed and with the canister housing in its intermediate fully-extended position relative to the expansion chamber and just prior to pivoting of same into its operable position;
FIG. 3 is a side elevational view of the device of Figs. 1 and 2 with the mouthpiece cap removed and with the canister housing pivoted into its operable position;
FIG. 4 is an end elevational view of the device as shown in Fig. 3 and taken generally along the line 4-4 of Fig. 3;
FIG. 5 is a longitudinal sectional view taken generally along the line 5-5 of Fig. 4;
FIG. 6 is a longitudinal sectional view taken generally along the line 6-6 of Fig. 3 but with the device in its intermediate fully-extended position of Fig. 2;
FIG. 7 is a fragmentary sectional view taken generally along line 7-7 of Fig. 6 but with the valve stem housing and canister supporting ribs not shown; and
FIG. 8 is an end elevational view taken generally along line 8-8 of Fig. 3 but with the expansion chamber not shown.

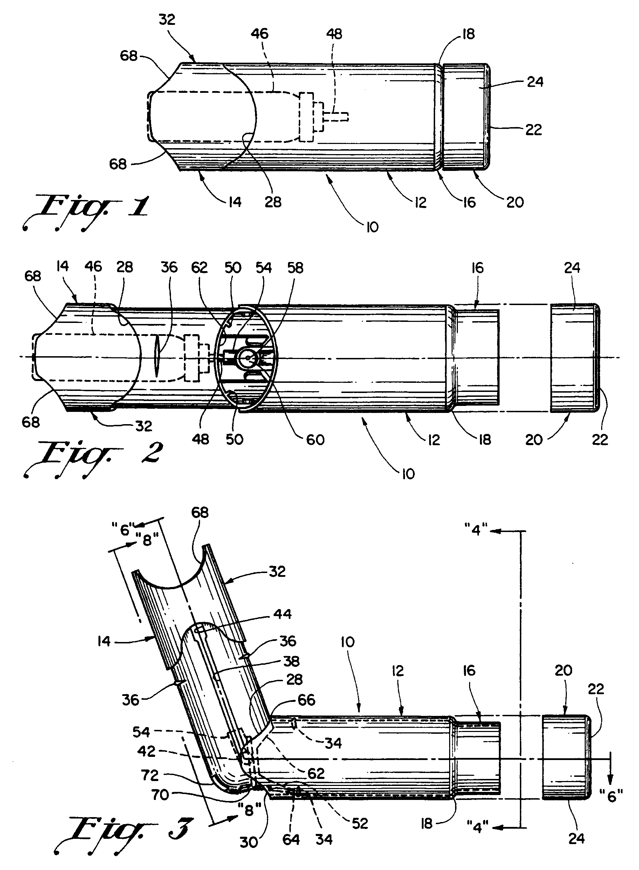

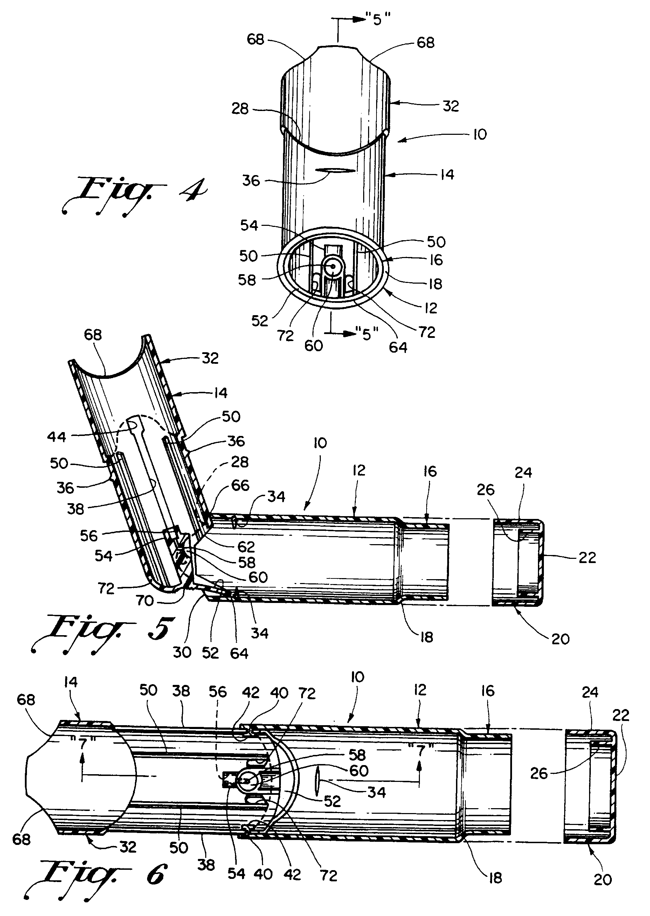

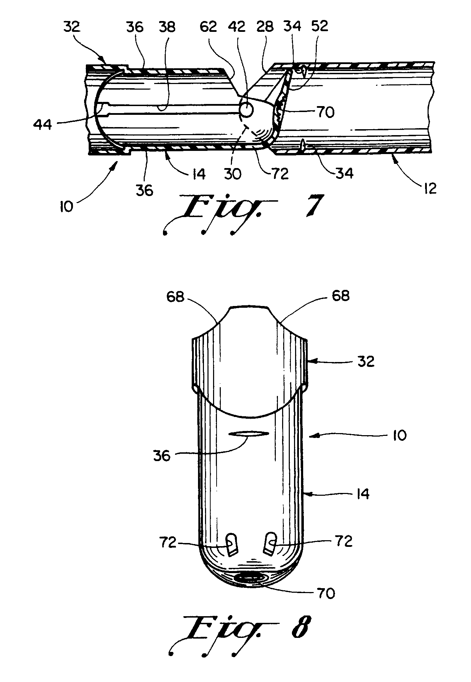

A preferred embodiment of the present invention comprises a compact, pocket or purse-size aerosol inhalation device **10**, as illustrated in Fig. 1, which opens up into an operable mode, as illustrated in Fig. 3. The device **10** comprises an elongated expansion chamber **12** elliptical in cross-section, a mouthpiece configuration **16** provided at one end of said expansion chamber **12**, an elongated canister housing **14** telescopically receivable in said expansion chamber **12** and open at one end for receiving therein a pressurized canister **46** of medicament.

Expansion chamber **12**, canister housing **14,** and mouthpiece cap **20** are all molded of appropriate plastic materials. At the open end of the expansion chamber **12** opposite the mouthpiece end thereof, the upper and lower wall portions are scooped inwardly, as at the generally concave rear edges **28** and **30** respectively, thereof, for purposes that will be discussed hereinafter. However, to provide a smooth-walled pocket or purse aerosol inhalation device **10** when the canister housing **14** is in its storage mode telescopically received within the expansion chamber **12,** the canister housing **14** is provided at its outer end with a saddle-shaped outwardly offset wall portion **32** having a configuration which complements and mates with the rear end configuration of the expansion chamber **12**. Detent means in the form of shallow upper and lower transverse grooves **34** formed on the inner surface of the expansion chamber **12** and transverse ridges **36** provided on the non-offset upper and lower side walls of the canister housing **14** serve to retain the canister housing **14** in its Fig. 1 storage position.

Pin and slot means are provided for connecting the canister housing **14** to the expansion chamber **12** with a pair of diametrically opposite, longitudinally extending slots **38** being provided in the side walls of the canister housing **14** and with a pair of diametrically opposite pins **40** projecting inwardly from the rear ends of the non-scooped side walls of the expansion chamber **12** and through the slots **38.** The pins **40** have enlarged heads **42** for retaining the pins **40** in the slots **38** with the rear ends of the slots **38** being enlarged, as at **44** to facilitate assembly of the canister housing **14** to the expansion chamber **12.**

The rear end of the canister housing **14** is open to permit insertion therein of pressurized canisters **46** of suitable medicaments for the treatment of lung disorders, such as peptides, an example of which is leuprolide. The canister **46** is of a known type having a hollow valve stem **48** which is biased outwardly of the front end of the canister **46** in axial alignment with the longitudinal axis thereof. The canister **46** is centered in the housing **14** by a series of four longitudinally extending ribs **50** which support said pressurized canister **46** of medicament therein.

Said canister housing **14** has a valve-stem-receiving portion **54** which is provided with an axial bore **56** which is axially aligned with the longitudinal axis of the canister housing **14** and is adapted to sealingly receive the valve stem **48** of the pressurized canister **46** therein. A laterally disposed orifice passage **58** is formed in the valve stem housing portion **54** and intersects the axial horn **56** therein at an obtuse first angle of approximately 110° or a conversely acute first angle of approximately 70°. The exit end of the passageway **58** is flared or generally conical in configuration as at **60**, and faces generally upwardly of the canister housing **14** as viewed in Figs. 2 and 6. An upper side wall portion of the canister housing **14** opposite the flared exit end **60** of the orifice passage **58** is either broken or cut away or left open during the molding process, as at an edge **62,** for reasons that will become obvious hereinafter.

To use the aerosol inhalation device **10** of the present invention when same is in its storage mode as shown in Fig. 1 with a pressurized canister **46** of medicament disposed within the elliptical canister housing **14,** with the elliptical canister housing **14** being telescopically retained within the elliptical expansion chamber **12,** and with the cap **20** mounted on the mouthpiece configuration **16** the cap **20** is first removed and the canister housing **14** is slidably moved to its fully-extended position generally axially aligned with the expansion chamber **12**, as shown in Figs . 2 and 6, the pins **40** being in engagement with the non-enlarged inner ends of the slots **38.**

The upper and lower side wall scooped out portions at **26** and **28**, of the expansion chamber **12** and the cut-away upper side wall portion, at **62,** of the canister housing **14** permit pivoting movement of the canister housing **14** relative to the expansion chamber **12** into the operable mode or position shown in Figs. 3, 4, 5 and 8 of the drawings wherein the obtuse second angle therebetween is approximately 110° and with the acute second angle between the longitudinal axes thereof heing approximately 70°. This pivoting second angle between the expansion chamber **12** and the canister housing **14** in the operable mode of the aerosol inhalation device **10** is pre-determined by interference contact between 1) the forward edge of the curved end wall **52** of the canister housing **14** with the inner surface of the lower side wall of the expansion chamber **12** , as at **64** in Figs. 3 and 5, and 2) the upper side wall of the canister housing **14** adjacent to the edge **62** thereof with the rear edge **28** of the inward portion of the upper side wall of the expansion chamber **12**, as is also best illustrated at **66** in Figs. 3 and 5.

From the pressurized canister **46** and through the orifice passage **58** into the elliptical expansion chamber **12,** the movement of the aerosolized medicament with this structural arrangement is a vortex movement which minimizes aggregation of the medicament particles and slows down their movement so as to minimize impaction of same against the back of the user's throat while maximizing the amount of the medicament delivered to the deepest portions of the lungs.

Although the obtuse second angle between the canister housing **14** and the expansion chamber **12** in the operable mode of the preferred embodiment of the device **10** disclosed herein is 110°, it is noted that other obtuse second angles could come within the scope of the invention, for instance second angles from 135° to 90°, as long as the first angle between the axis of the orifice passage **58** and the longitudinal axis of the canister housing **14** is such that in the operable position of the device the axis of the orifice passage **58** is axially aligned with the longitudinal axis of an elliptical cross-section expansion chamber.

It is noted that for most effective operation of this aerosol inhalation device **10**, auxiliary air inlet ports **72** are provided in the lower side wall of the canister housing **14** rearwardly of the flared exit end **60** of the orifice passage **58** and on opposite sides thereof as is best illustrated in Figs. 2, 3, 4, 6 and 8.

As shown in Figs. 1 and 2, the rear end of the pressurized canister of medicament **46** is approximately flush with the rear edge of the canister housing **14** when inserted therein. This arrangement minimizes inadvertent actuation of the canister **46** and unintended release of the medicament. As an aid to activating the canister **46** to release medicament therefrom by moving same inwardly of the housing **14**, the side edges of the housing **14** are cut away inwardly, as at **68,** to facilitate inward movement of a finger engageable with the end of the canister **46.**

As a further aid to actuating the device **10,** a thumbreceiving depression **70,** which may be roughened or knurled, is provided in the outer surface of the inner end wall **52** of the canister housing **14.**

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. An aerosol inhalation device (10) for maximizing delivery of medicament into the lung, said device (10) comprising an elongated expansion chamber (12) with a mouthpiece configuration (16) provided at one end thereof, an elongated canister housing (14) associated with said expansion chamber (12) for receiving a pressurized canister (46) of medicament having a valve stem (48) projecting from one end thereof, said canister housing (14) being telescopically slidable into said expansion chamber (12) and said canister housing (14) being pivotally connected to said expansion chamber (12) by connecting means further permitting axial movement of said canister housing (14) from a storage position telescoped within said expansion chamber (12) to an axially-aligned, fully-extended position, said canister housing (14) being pivotal only when in said fully-extended position into an operable position, said operable position being predetermined by interference means between said expansion chamber (12) and said canister housing (14), thereby limiting said relative pivotal movement therebetween, said canister housing (14) having a valve-stem-receiving portion (54), a laterally disposed orifice passage (58) formed in said valve-stem-receiving portion (54) and intersecting a valve-stem-receiving bore (56) thereof with the axis of said orifice passage (58) being disposed at a first angle to the axis of said valve-stem-receiving bore (56) and with an exit end (60) of said orifice passage (58) being generally flared outwardly, said canister housing (14) being pivotal only through a second angle equal to or less than 90° into said operable position wherein the axis of said orifice passage (58) is disposed in axial alignment with a longitudinal axis of said elliptical expansion chamber (12), said first angle between said orifice passage (58) and said axis of said valve-stem-receiving-bore (56) being equal to or greater than 90°,
characterized in that said expansion chamber (12) is elliptical in cross section.

2. The aerosol inhalation device (10) according to claim 1, wherein said canister housing (14) is also elliptical in cross section.

3. The aerosol inhalation device (10) according to claim 1 or 2, wherein an air inlet means (72) is provided in said canister housing (14) adjacent said orifice passage (58).

4. The aerosol inhalation device (10) according to one or more of the preceding claims, wherein said interference means (52) are also adapted to limit telescopic movement of said canister housing (14) into said storage position within said expansion chamber (12).

5. The aerosol inhalation device (10) according to one or more of the preceding claims, wherein a mouthpiece configuration (16) having lip-engageable shoulder means is provided at one end of said expansion chamber (12) and wherein a removable cap (20) is provided for said mouthpiece configuration (16).

6. The aerosol inhalation device (10) according to one or more of the preceding claims, wherein said connecting means is characterized by pin means (40) provided on said expansion chamber and by slot means (38) provided in said canister housing (14).

7. The aerosol inhalation device (10) of one or more of the preceding claims, wherein said first angle between the axis of the orifice passage(58) and the axis of the valve-stem-receiving bore(56) is approximately 110° and wherein said second angle through which said canister housing (14) is pivotal relative to said expansion chamber (12) is approximately 70°.

8. The aerosol inhalation device (10) according to one or more of the preceding claims, wherein longitudinally extending, circumferentially spaced rib members (56) are provided in said canister housing (14) for supporting said canister (46) of medicament therein.

9. The aerosol inhalation device (10) according to one or more of the preceding claims, wherein finger-and-thumb engageable depressions (70) are provided at opposite ends of said canister housing (14) as an aid in releasing medicament from said pressurized canister (46) thereof.

10. The aerosol inhalation device (10) according to one or more of the preceding claims, wherein a side wall portion of said canister housing opposite said orifice passage (58) is broken away.

11. The aerosol inhalation device (10) according to one or more of the preceding claims, wherein the side edges of said canister housing (14) are cut away inwardly (68) to facilitate inward movement of a finger engageable with the end of said canister (46).

## Patentansprüche

1. Eine Aerosol-Inhalationsvorrichtung (10) zur Maximierung der Verabreichung eines Medikaments in die Lunge, wobei die Vorrichtung (10) eine längliche Expansionskammer (12) mit einer Mundstückausgestaltung (16) an einem Ende davon umfaßt, und ein längliches Behältergehäuse (14) umfaßt, das mit der Expansionskammer (12) verbunden ist, um einen druckbeaufschlagten Behälter (46) mit einem Medikament aufzunehmen, der über einen ventilschaft (48) verfügt, der sich aus einem Ende davon erstreckt, wobei das Behältergehäuse (14) teleskopisch in die Expansionskammer (12) verschoben werden kann und wobei das Behältergehäuse (14) durch ein Verbindungsmittel drehbar mit der Expansionskammer (12) verbunden wird, das weiterhin die axiale Bewegung des Behältergehäuses (14) aus einer Lagerungsstellung, in der das Behältergehäuse innerhalb der Expansionskammer (12) teleskopartig eingerückt ist, in eine axial ausgerichtete, vollständig auseinandergezogene Stellung erlaubt, wobei das Behältergehäuse (14) nur dann in die Betriebsstellung gedreht werden kann, wenn es sich in der vollständig auseinandergezogenen Stellung befindet, wobei die Betriebsstellung durch das Eingriffsmittel zwischen der Expansionskammer (12) und dem Behältergehäuse (14) vorbestimmt wird, wodurch die relative Drehbewegung dazwischen eingeschränkt wird, wobei das Behältergehäuse (14) über einen Ventilschaft-Aufnahmeabschnitt (54) verfügt, wobei ein seitlich angeordneter Mündungsdurchlaß (58) der im Ventilschaft-Aufnahmeabschnitt (54) ausgebildet ist und eine Ventilschaft-Aufnahmebohrung (56) davon mit der Achse des Mündungsdurchlasses (58) schneidet, in einem ersten Winkel zur Achse der Ventilschaft-Aufnahmebohrung (56) angeordnet ist, und wobei ein Auslaßende (60) des Mündungsdurchlasses (58) allgemein außen erweitert ist, wobei das Behältergehäuse (14) lediglich bis einen zweiten Winkel, der gleich oder kleiner als 90° ist, in die Betriebsstellung gedreht werden kann, worin die Achse des Mündungsdurchlasses (58) in axialer Ausrichtung mit einer Längsachse der elliptischen Expansionskammer (12) angeordnet ist, wobei der erste Winkel zwischen dem Mündungsdurchlaß (58) und der Achse der Ventilschaft-Aufnahmebohrung (56) gleich oder größer als 90° ist,
dadurch gekennzeichnet, daß die Expansionskammer (12) im Querschnitt elliptisch ist.

2. Die Aerosol-Inhalationsvorrichtung (10) nach Anspruch 1, worin das Behältergehäuse (14) im Querschnitt ebenfalls elliptisch ist.

3. Die Aerosol-Inhalationsvorrichtung (10) nach Anspruch 1 oder 2, worin ein Luft-Einlaßmittel (72) im Behältergehäuse (14) angrenzend am Mündungsdurchlaß (58) bereitgestellt wird.

4. Die Aerosol-Inhalationsvorrichtung (10) nach einem oder mehreren der vorangegangenen Ansprüche, worin das Eingriffsmittel (52) ebenso dazu ausgebildet ist, die teleskopische Bewegung des Behältergehäuses (14) in die Lagerungsstellung innerhalb der Expansionskammer (12) zu begrenzen.

5. Die Aerosol-Inhalationsvorrichtung (10) nach einem oder mehreren der vorangegangenen Ansprüche, worin eine Mundstückausgestaltung (16), die eine mit einer Lippe in Eingriff bringende Schulter aufweist, an einem Ende der Expansionskammer (12) bereitgestellt wird und worin eine abnehmbare Kappe (20) für die Mundstückausgestaltung (16) bereitgestellt wird.

6. Die Aerosol-Inhalationsvorrichtung (10) nach einem oder mehreren der vorangegangenen Ansprüche, worin das Verbindungsmittel durch einen Stift (40), der an der Expansionskammer bereitgestellt wird, und durch einen Schlitz (38), der im Behältergehäuse (14) bereitgestellt wird, gekennzeichnet ist.

7. Die Aerosol-Inhalationsvorrichtung (10) nach einem oder mehreren der vorangegangenen Ansprüche, worin der erste Winkel zwischen der Achse des Mündungsdurchlasses (58) und der Achse der Ventilschaft-Aufnahmebohrung (56) annähernd 110° beträgt und worin der zweite Winkel, über den das Behältergehäuse (14) in bezug auf die Expansionskammer (12) gedreht werden kann, annähernd 70° beträgt.

8. Die Aerosol-Inhalationsvorrichtung (10) nach einem oder mehreren der vorangegangenen Ansprüche, worin die sich länglich erstreckenden, am Umfang entlang beabstandeten Rippenglieder (56) im Behältergehäuse (14) bereitgestellt werden, um darin den Behälter (46) mit dem Medikament zu stützen.

9. Die Aerosol-Inhalationsvorrichtung (10) nach einem oder mehreren der vorangegangenen Ansprüche, worin als Hilfe zur Freigabe des Medikaments aus dem druckbeaufschlagten Behälter (46) eingreifbare Vertiefungen (70), zu denen man mit dem Finger und Daumen Zugriff hat, an den gegenüberliegenden Enden des Behältergehäuses (14) bereitgestellt werden.

10. Die Aerosol-Inhalationsvorrichtung (10) nach einem oder mehreren der vorangegangenen Ansprüche, worin ein Seitenwandabschnitt des Behältergehäuses gegenüber dem Mündungsdurchlaß (58) weggebrochen ist.

11. Die Aerosol-Inhalationsvorrichtung (10) nach einem oder mehreren der vorangegangenen Ansprüche, worin die Seitenkanten des Behältergehäuses (14) nach Innen abgeschnitten sind (68), um die Innenbewegung eines Fingers, der in das Ende des Behälters (46) eingreifen kann, zu erleichtern.

## Revendications

1. Dispositif d'inhalation d'aérosol (10) pour maximiser la délivrance de médicaments dans le poumon, ledit dispositif (10) comprenant une chambre de detente allongée (12) avec une configuration d'embout buccal (16) prévue à une extrémité de celle-ci, et un boîtier de récipient métallique allongé (14) associé avec ladite chambre de détente (12) pour recevoir un récipient métallique de médicament sous pression (46) ayant une tubulure de valve (48) qui fait saillie depuis l'une de ses extrémités, ledit boîtier de récipient métallique (14) pouvant coulisser de manière télescopique dans ladite chambre de détente (12) et ledit boîtier de récipient métallique (14) étant relié à pivotement à ladite chambre de détente (12) par des moyens de liaison permettant en outre un mouvement axial dudit boîtier de récipient métallique (14) depuis une position de stockage télescopique à l'intérieur de ladite chambre de détente (12) jusqu'à une position totalement étendue, alignée axialement, ledit boîtier de récipient métallique (14) pouvant pivoter dans une position actionnable, seulement lorsqu'il est dans ladite position totalement étendue, ladite position actionnable étant prédéterminée par des moyens d'interférence entre ladite chambre de détente (12) et ledit boîtier de récipient métallique (14), ce qui limite ledit mouvement de pivotement relatif entre eux, ledit boîtier de récipient métallique (14) ayant une partie de réception de tubulure de valve (54), un passage d'orifice (58) disposé latéralement formé dans ladite partie de réception de tubulure de valve (54) et rencontrant un alésage de réception de tubulure de valve (56) de celle-ci, l'axe dudit passage d'orifice (58) étant disposé sous un premier angle par rapport à l'axe dudit alésage de réception de tubulure de valve (56) et une extrémité de sortie (60) dudit passage d'orifice (58) étant généralement évasée vers l'extérieur, ledit boîtier de récipient métallique (14) pouvant pivoter seulement sur un second angle égal ou inférieur à 90° dans ladite position actionnable où l'axe dudit passage d'orifice (58) est disposé en alignement axial avec un axe longitudinal de ladite chambre de détente elliptique (12), ledit premier angle entre ledit passage d'orifice (58) et ledit axe dudit alésage de réception de tubulure de valve (56) étant égal ou supérieur à 90°,
caractérisé en ce que ladite chambre de détente (12) est de section droite elliptique.

2. Dispositif d'inhalation d'aérosol (10) selon la revendication 1, où ledit boîtier de récipient métallique (14) est aussi de section droite elliptique.

3. Dispositif d'inhalation d'aérosol (10) selon la revendication 1 ou 2, où un moyen d'entrée d'air (72) est prévu dans ledit boîtier de récipient métallique (14) en position adjacente audit passage d'orifice (58).

4. Dispositif d'inhalation d'aérosol (10) selon une ou plusieurs des revendications précédentes où lesdits moyens d'interférence (52) sont conçus aussi pour limiter le mouvement télescopique dudit boîtier de récipient métallique (14) dans ladite position de stockage à l'intérieur de ladite chambre de détente (12).

5. Dispositif d'inhalation d'aérosol (10) selon une ou plusieurs des revendications précédentes où une configuration d'embout buccal (16) ayant des moyens formant épaulements pouvant coopérer avec des lèvres est prévue à une extrémité de ladite chambre de détente (12) et où un capuchon amovible (20) est prévu pour ladite configuration d'embout buccal (16).

6. Dispositif d'inhalation d'aérosol (10) selon une ou plusieurs des revendications précédentes, où ledit moyen de liaison est caractérisé par des moyens formant ergots (40) prévus sur ladite chambre de détente et par des moyens formant fentes (38) prévus dans ledit boîtier de récipient métallique (14).

7. Dispositif d'inhalation d'aérosol (10) selon une ou plusieurs des revendications précédentes où ledit premier angle entre l'axe du passage d'orifice (58) et l'axe de l'alésage de réception de tubulure de valve (56) est d'environ 110° et où ledit second angle suivant lequel ledit boîtier de récipient métallique (14) peut pivoter par rapport à ladite chambre de détente (12) est d'environ 70°.

8. Dispositif d'inhalation d'aérosol (10) selon une ou plusieurs des revendications précédentes, où des éléments formant nervures (56) qui s'étendent longitudinalement et qui sont répartis sur la circonférence sont prévus dans ledit boîtier de récipient métallique (14) pour soutenir à l'intérieur ledit récipient métallique (46) de médicament.

9. Dispositif d'inhalation d'aérosol (10) selon une ou plusieurs des revendications précédentes où des évidements (70) qui peuvent coopérer avec un doigt et un pouce sont prévus au niveau d'extrémités opposées dudit boîtier de récipient métallique (14) en tant qu'aide pour libérer le médicament depuis son récipient métallique sous pression (46).

10. Dispositif d'inhalation d'aérosol (10) selon une ou plusieurs des revendications précédentes, où une partie de paroi latérale dudit boîtier de récipient métallique opposée audit passage d'orifice (58) est interrompue.

11. Dispositif d'inhalation d'aérosol (10) selon une ou plusieurs des revendications précédentes, où les bords latéraux dudit boîtier de récipient métallique (14) sont découpés vers l'intérieur (68) pour faciliter le mouvement vers l'intérieur d'un doigt qui peut coopérer avec l'extrémité dudit récipient métallique (46).
